# EUROPÄISCHE PATENTSCHRIFT

(11) **EP 1 753 510 B1**
(45) Veröffentlichungstag und Bekanntmachung des Hinweises auf die Patenterteilung: **25.08.2010**
(21) Anmeldenummer: 05759699.1
(22) Anmeldetag: 09.06.2005
(51) Int. Cl.: A61N 5/10

(54) **VORRICHTUNG ZUR KOMPENSATION VON BEWEGUNGEN EINES ZIELVOLUMENS WÄHREND EINER IONENSTRAHL-BESTRAHLUNG**
DEVICE FOR COMPENSATING MOVEMENTS OF A TARGET VOLUME DURING ION BEAM IRRADIATION
DISPOSITIF DE COMPENSATION DES DEPLACEMENTS D'UN VOLUME CIBLE PENDANT UNE IRRADIATION PAR FAISCEAU IONIQUE

(30) Priorität: 09.06.2004 DE 102004028035
(43) Veröffentlichungstag der Anmeldung: 21.02.2007
(73) Patentinhaber: GSI Helmholtzzentrum für Schwerionenforschung GmbH, 64291 Darmstadt (DE)
(72) Erfinder: GROEZINGER, Sven, Oliver, 64625 Bensheim (DE); HABERER, Thomas, 60529 Frankfurt (DE); OTT, Wolfgang, 69221 Dossenheim (DE); POPPENSIEKER, Klaus, 64287 Darmstadt (DE)
(74) Vertreter: Forstmeyer, Dietmar
(86) Internationale Anmeldenummer: PCT/EP2005/006224
(87) Internationale Veröffentlichungsnummer: WO 2005/120641

(56) Entgegenhaltungen:
- WO-A-00/49624
- WO-A-00/59575
- US-A1- 2003 136 924
- US-B1- 6 405 072
- BRAHME A.: "Recent advances in light ion radiation therapy" INT. J. RADIATION ONCOLOGY BIOL. PHYS., Bd. 58, Nr. 2, 1. Februar 2004 (2004-02-01), Seiten 603-616, XP002351898

## Beschreibung

Die vorliegende Erfindung betrifft eine Vorrichtung zur Kompensation von dreidimensionalen Bewegungen eines Zielvolumens auf einer Patientenliege während einer Ionenstrahl-Bestrahlung insbesondere mit einer Rasterscanvorrichtung. Diese Vorrichtung umfasst ein Positionslage- und Verfolgungssystem, das die dreidimensionalen Bewegungen des Zielvolumens longitudinal und transversal zum Ionenstrahl erfasst. Ferner weist die Vorrichtung einen Tiefenmodulator auf, mit dem die Eindringtiefe des Ionenstrahls nachgeregelt werden kann. Außerdem steht in einer Weiterbildung die Vorrichtung mit der Rasterscanvorrichtung, die eine transversale Ablenkung des Ionenstrahls in Bruchteilen von Millisekunden ermöglicht in Wirkverbindung.

Eine derartige Vorrichtung ist aus der Druckschrift DE 100 31 074 A1 bekannt, in der auch das Verfahrensprinzip zur Präzisionsbestrahlung von bewegten Zielvolumina mit dem Ionen-Rasterscanverfahren vorgestellt wird. Die beschriebene Methode erfordert eine dynamische Korrektur der transversalen und longitudinalen Bestrahlungsparameter zur Laufzeit der Bestrahlung.

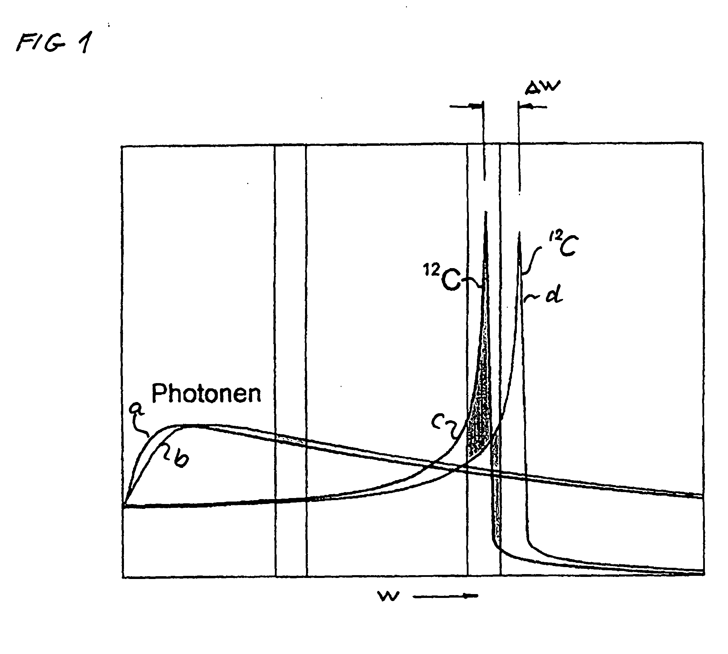

Die transversale Korrektur basiert auf der Rasterscanmethode . Mit dem Rasterscanverfahren können somit die Einstellungen in transversaler Richtung in Bezug auf den Ionenstrahl von Bestrahlungspunkt zu Bestrahlungspunkt geändert und damit korrigiert werden. Da für das Rasterscanverfahren auch eine Intensitätssteuerung vorgesehen ist, ermöglicht diese die Anpassung auch der longitudinalen Bestrahlungsparameter einer im Voraus bestimmten Sollposition. Während die transversalen Anpassungen und Korrekturen in Bruchteilen von Millisekunden erfolgen können, sind jedoch die longitudinalen Einstellungen nur synchron zum Zyklus des Teilchenbeschleunigers veränderbar und damit sehr langsam. Das damit verbundene Problem in Bezug auf die Eindringtiefe des Ionenstrahls in das Gewebe wird in Figur 1 verdeutlicht.

Figur 1 zeigt die Auswirkungen von Änderungen der Konsistenz der Struktur eines gesunden Gewebes, das strahlaufwärts das Zielvolumen bedeckt auf die Dosisverteilung bei einer Photonenbestrahlung mit den Kurven a und b im Vergleich zu einer Ionenbestrahlung mit den Kurven c und d. Dazu ist auf der Abszisse die Eindringtiefe w aufgetragen und auf der Ordinate die vom Gewebe aufgenommene Ionendosis. Mit einer dreidimensionalen Bewegung eines Körpers eines Patienten auf einer Patientenliege ändert sich nämlich nicht nur die Lage des Patienten und damit die Lage des Zielvolumens transversal und longitudinal zum Ionenstrahl, sondern auch die Zusammensetzung, die Dichte, die Dicke und die Konsistenz des strahlaufwärts des Zielvolumens angeordneten gesunden Gewebes, was zu der veränderten Kurve b (während oder nach einer Bewegung) gegenüber der Kurve a (vor der Bewegung) in der Dosisverteilung bei Photonenbestrahlung führt.

Bei der Ionenbestrahlung sind die Auswirkungen wesentlich gravierender, wie der Vergleich der Kurven c (vor der Bewegung) und d (während oder nach einer Bewegung) in Figur 1 zeigt, da bei der Ionenbestrahlung kein exponentielles Abklingen der Dosisverteilung mit der Eindringtiefe wie bei der Photonenbestrahlung auftritt, sondern eine Dosiseskalation, die sich bei Bewegung beispielsweise um die Eindringtiefendifferenz Δw aufgrund des sich ändernden Abdeckgewebes verschieben kann und somit vom geplanten Volumenelement des Zielvolumens abweicht.

Im Gegensatz zu Photonen ist die durch Ionenbestrahlung applizierte Dosisverteilung demnach äußerst sensitiv auf Änderungen in longitudinaler Richtung, bspw. bei Dichteänderungen, im durchstrahlten gesunden Gewebe. Solche Änderungen treten bspw. bei der atmungsbedingten Organbewegung auf einer kürzeren Zeitscala als der Beschleunigerzyklus auf, mit dem das herkömmliche Rasterscanverfahren reagieren könnte. Das bedeutet, dass eine Korrektur, auch der Eindringtiefen, Änderungen durch eine Bewegung des Patienten in einer kurzen Zeitspanne von Bestrahlungsposition zu Bestrahlungsposition mit dem herkömmlichen Rasterscanverfahren nicht exakt verfolgen kann. Ein Erfassen der longitudinalen Verschiebung des Zielvolumens beim Bewegen des Patienten allein durch Präzisionsvideokameras, wie es aus der Druckschrift DE 100 31 074 A1 bekannt ist, lässt keine exakte Strahlanpassung oder Korrektur zu, selbst wenn diese Anpassung von Strahlposition zu Strahlposition aufgrund der dort offenbarten Vorrichtung für die Verlagerung des Zielvolumens erfolgen könnte.

Das intensitätsmodulierte Rasterscanverfahren ermöglicht die Bestrahlung von tief liegenden Tumoren mit extrem hoher geometrischer Präzision, jedoch relativ langsam. Der Therapieerfolg ist jedoch bei Strahlentherapie abhängig von der Dosis im Zielvolumen. Diese wird in der Regel beschränkt durch die Toleranzdosen im umliegenden Gewebe. Im Vergleich zu einer konventioneller Photonenbestrahlung ermöglicht die geometrische Präzision des intensitätsmodulierten Rasterscanverfahrens in vielen Fällen eine Dosiseskalation im Zielvolumen, wie es Figur 1 zeigt. Um diese Präzision nutzen zu können, muss jedoch während der Bestrahlung die relative Positionslage des Zielvolumens jederzeit mit dem in einer Bestrahlungsplanung angenommenen Fall übereinstimmen. Die routinemäßig verwendete Fixierung des Zielgebietes liefert nicht in allen Fällen, bspw. im Thoraxbereich, eine ausreichende Genauigkeit. Jede verbleibende Längenänderung oder Verschiebung des zu bestrahlenden Volumens relativ zu der Referenzposition für die Bestrahlungsplanung führt zu einer Fehlpositionierung und damit zu einer Fehldosierung von Ionen. Somit stimmt die tatsächlich applizierte Ionenanzahl pro Volumen nicht mehr mit der geplanten Verteilung überein, d.h. die Homogenität und Geometrie der Dosisverteilung ändert sich und der Therapieerfolg wird dadurch gefährdet.

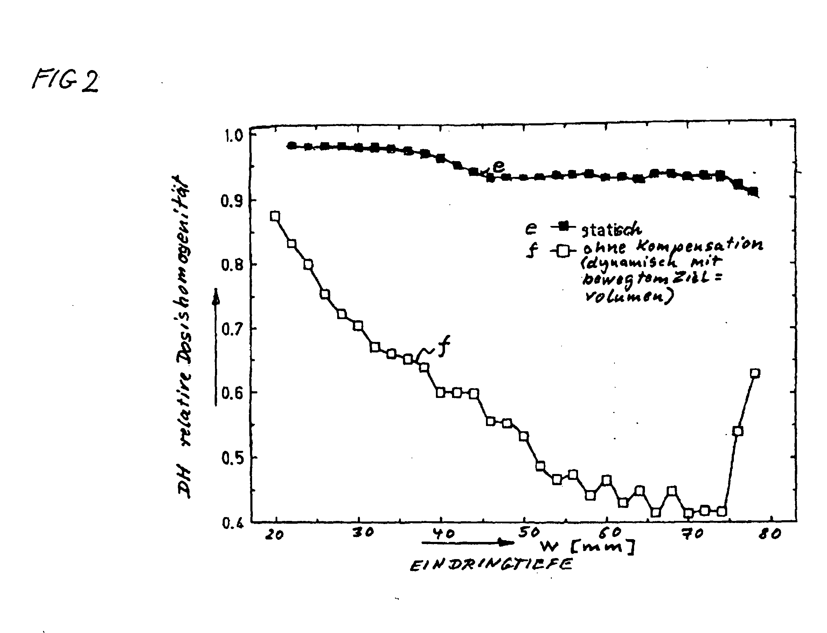

Dieses Problem verdeutlicht Figur 2, welche die relative Dosis Homogenität für ein statisch fixiertes Zielvolumen mit der Kurve e und für ein lageverändertes Zielvolumen mit der Kurve f in Abhängigkeit von der Eindringtiefe w darstellt. Selbst bei einem statischen bzw. fixierten Zielvolumen nimmt die Dosishomogenität mit zunehmender Eindringtiefe w aufgrund von Streu- und Absorbtionsmechanismen des Ionenstrahls im Gewebe ab. Diese Abnahme bei statischer, d.h. fixierter Lage des Zielvolumens beträgt jedoch nicht mehr als 10%

(Kurve e, Figur 2) der eingestrahlten Dosis. Bei einer Bewegung des Zielvolumens können jedoch, wie bereits Figur 1 zeigt, erhebliche longitudinale Eindringtiefenänderungen auftreten, sodass sich die relative Dosishomogenität, wie Figur 2 zeigt, bis zu 60% verschlechtern kann (Kurve f, Figur 2), wenn keine Kompensation erfolgt.

Aufgabe der Erfindung ist es, eine Vorrichtung zur Kompensation von Bewegungen eines Zielvolumens während einer Ionenstrahl-Behandlung anzugeben, das die oben beschriebenen Probleme überwindet und die Präzision der Bestrahlung eines Zielvolumens weiter verbessert.

Diese Aufgabe wird mit dem Gegenstand der unabhängigen Ansprüche gelöst. Vorteilhafte Weiterbildungen der Erfindung ergeben sich aus den abhängigen Ansprüchen.

Erfindungsgemäß wird eine Vorrichtung zur Kompensation von dreidimensionalen Bewegungen eines Zielvolumens auf einer Patientenliege während einer Ionenstrahl-Bestrahlung mit einer Rasterscanvorrichtung geschaffen. Dazu weist die Kompensationsvorrichtung ein Positionslage- und Verfolgungssystem auf, das die dreidimensionalen Bewegungen des Zielvolumens longitudinal und transversal zum Ionenstrahl erfasst und dabei mit einem Bewegungsmessungs-, Steuer- und Auslesemodul SAMB in Wirkverbindung steht. Ferner weist die Kompensationsvorrichtung einen Tiefenmodulator auf, der mit dem Bewegungsmessungs-, Steuer- und Auslesemodul SAMB zur Änderung der Eindringtiefe w des Ionenstrahls in Wirkverbindung steht. Außerdem steht die Rasterscanvorrichtung als Teil der Vorrichtung, die den Ionenstrahl transversal ablenkt, mit einem Ortsmessungs-, Steuer- und Auslesemodul SAMO zur Änderung der Strahlauslenkung in transversaler Richtung in Wirkverbindung.

Dazu weist das Bewegungsmessungs-, Steuer- und Auslesemodul SAMB einen Mikroprozessor mit Speicher auf. Der Speicher enthält Daten eines Modells einer Struktur des gesunden Gewebes, das strahlaufwärts das Zielvolumen bedeckt. Der Mikroprozessor weist außerdem Rechenkomponenten auf, welche die erfassten Bewegungen des Zielvolumens vektoriell in longitudinale und transversale Anteile gliedern. Ferner gleichen die Rechenkomponenten die longitudinalen Anteile am gespeicherten Modell zur Korrektur der Eindringtiefe des Ionenstrahls ab.

Vorzugsweise sind die Daten eines Modells in dem Speicher von Ultraschallschnitten oder Röntgenbildern des gesunden Deckgewebes über dem Zielvolumen abgeleitet. Sowohl Röntgenals auch Ultraschalluntersuchungen im Vorfeld der Ionenbestrahlung haben den Vorteil, dass sie das gesunde Deckgewebe über dem Zielvolumen, sowohl in ihrer Dicke als auch in ihrer Zusammensetzung, ihrer Konsistenz und ihrer Dichte exakt wiedergeben können. Damit wird die Präzision der Kompensation longitudinaler Abweichungen wesentlich präziser durchführbar als bei herkömmlichen Vorrichtungen und Verfahren. Zusätzlich lässt sich durch weitere Messungen mittels Röntgenerfassungsgeräten und/oder Ultraschallerfassungsgeräten die Gültigkeit des Modells ständig gewährleisten.

Diese Vorrichtung hat darüber hinaus den Vorteil, dass aus der erfassten Lage- und Positionsänderung des aktuellen Zielvolumens zum Zeitpunkt der Bestrahlung sich ein dreidimensionaler Korrekturvektor für die radiologische Position des jeweiligen Volumenelementes bestimmen lässt und durch eine Kompensation der störenden Bewegung mit einer entgegengerichteten Verschiebung des Therapiestrahls dafür gesorgt wird, dass die im Vorfeld der Bestrahlung optimierte Ionenanzahl gemäß einem Bestrahlungsplan an das jeweilige Volumenelement abgegeben wird. Für die Kompensation von Bewegung des Zielgebiets während der Bestrahlung erfolgt eine Nachführung des Therapiestrahls in allen drei Raumrichtungen. Die Aufteilung der Kompensation in einen transversalen und einen longitudinalen Anteil ermöglicht die Nachführung mithilfe der Rasterscanvorrichtung und mithilfe eines Tiefenmodulators.

Im Fall, dass sich das Zielvolumen aufgrund seiner anatomischen Anordnung nicht bewegt, aber Bewegungen des Patienten zu Änderungen im durchstrahlten gesunden Gewebe führen, ist nur eine longitudinale Kompensation notwendig, somit ist die Energie des Ionenstrahls entsprechend der Bewegung anzupassen, so dass sich die Reichweite des Ionenstrahls jeweils derart ändert, dass die Wechselwirkung mit dem Zielvolumenelement sichergestellt ist. Dieser Fall ergibt sich auch dann, wenn beispielsweise die Strahlnachführung in transversaler Richtung an die Bewegung des Zielvolumens durch eine Rasterscanvorrichtung in Echtzeit erfolgt und somit während der Bestrahlung sichergestellt ist, dass der Ionenstrahl dem sich bewegenden Zielvolumen folgt. Im mitbewegten Koordinatensystem ergibt sich dann wieder bei einer Relativbewegung des durchstrahlten Gewebes zum Zielvolumen die Notwendigkeit, die Eindringtiefe des Ionenstrahls an das jeweils zu durchstrahlende Gewebe anzupassen.

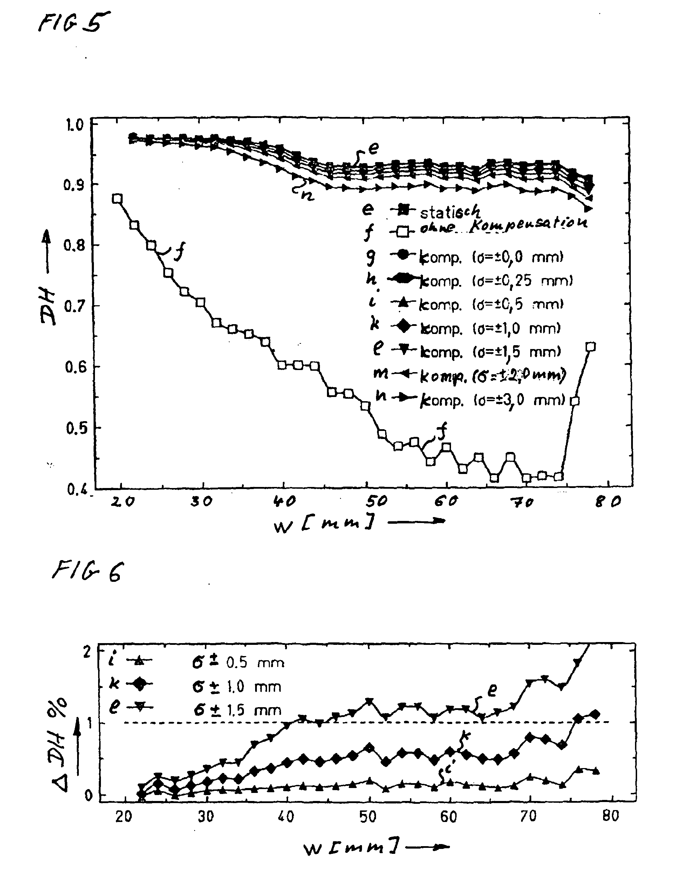

Die erzielbare Genauigkeit in der applizierten Dosisverteilung ist zwar von der Güte der Kompensation abhängig, wie es hiernach in Figur 5 und 6 gezeigt wird, doch prinzipiell sind große Homogenitäten erreichbar, die mit der Güte von statischen Zielvolumina aufgrund des Gegenstandes der vorliegenden Erfindung vergleichbar sind. Eine Präzisionsbestrahlung bewegter Zielgebiete bzw. bei sich bewegendem durchstrahltem Gewebe wird durch die sehr genaue Bewegungskompensation erreicht. Dazu korrigieren die Rasterscanvorrichtung und der Tiefenmodulator die Strahlpositionen während der Bestrahlung mit einer Geschwindigkeit, die deutlich größer ist als die Bewegung des Zielvolumens oder des durchstrahlten Gewebes.

Die korrigierte Position richtet sich nach der Sollposition im unkorrigierten Zustand und der aktuellen Verschiebung des jeweiligen Volumenelementes des Zielvolumens im Bezugssystem der Bestrahlungsplanung. Durch die feste Integration der Bewegungskompensation in das Kontrollsystem der Rasterscanvorrichtung wird ein Datenaustausch ermöglicht, der gewährleistet, dass die Sicherheit und Zuverlässigkeit der Dosisgabe pro Volumeneinheit des Zielvolumens trotz dreidimensionaler Bewegung verbessert wird. Somit ermöglicht der Gegenstand der vorliegenden Erfindung ein Ausweiten des bereits beschriebenen Rasterscanverfahrens auf Indikationen in Zielgebieten, die sich nicht oder nicht ausreichend fixieren lassen bzw. auf Zielgebiete, bei denen sich das durchstrahlte Gewebe durch Bewegung in seiner Energie absorbierenden Wirkung ändert. Er ermöglicht die Bestrahlung von Tumoren in Thorax und Abdomen mit einer ähnlich hohen Präzision, wie sie bei fixierten Zielgebieten erreichbar ist.

Bereits existierende Alternativen mit Bewegungskorrekturen führen entweder zu einer geringeren Präzision oder einer signifikant verlängerten Bestrahlungsdauer. Diese Methoden können die Aussicht auf eine erfolgreiche Therapie oder den Patientendurchsatz nachteilig reduzieren. Beide Konsequenzen treten bei dem vorgestellten Gegenstand der Erfindung nicht auf. Die erfindungsgemäße Vorrichtung kann ferner die Patientenpositionierung erleichtern, indem bei leichten Fehlpositionierungen automatisch die Strahlposition angepasst wird. Eine strenge Patientenfixierung ist mit der erfinderischen Vorrichtung nicht mehr zwingend erforderlich, wodurch der Patientenkomfort deutlich erhöht wird.

In einer bevorzugten Ausführungsform der Erfindung weist die Rasterscanvorrichtung zwei Rasterscanmagnete auf, die einen Ionenstrahl orthogonal in Bezug auf eine Einkopplungsrichtung in die Rasterscanmagnete in einer X- und einer Y-Richtung, die ihrerseits senkrecht zueinander liegen, zum scheibenweisen Flächenabtasten des Zielvolumens ablenken, wobei Rasterscanmagnete durch reaktionsschnelle Netzgeräte gesteuert werden. Das hat den Vorteil, dass eine transversale Kompensation der bewegungsbedingten transversalen Änderung des Zielvolumens und seines Deckgewebes von Bestrahlungsposition zu Bestrahlungsposition in Bruchteilen von Millisekunden erfolgen kann.

Vorzugsweise weist die Vorrichtung mindestens einen Beschleuniger auf, mit dem die Energie des Ionenstrahls einstellbar ist, sodass das Zielvolumen scheibenweise in der Eindringtiefe gestaffelt bestrahlbar ist. Damit ist der Vorteil verbunden, dass nacheinander das gesamte Zielvolumen scheibenweise abtastbar wird, wobei von Scheibe zu Scheibe die Reichweite des Ionenstrahls durch Änderung der Energie des Ionenstrahls einstellbar ist. Der Beschleuniger besteht zu diesem Zweck im Wesentlichen aus einem Linearbeschleuniger und/oder einem Synchrotron oder einem Zyklotron, in denen Protonen und oder schwere Ionen mit gleicher Masse in ihrer Energie stufenweise eingestellt werden können.

Die Energieanpassung des Ionenstrahls an vorgegebene Reichweiten innerhalb des Bestrahlungsraums, insbesondere innerhalb des Zielvolumens ist aufgrund der Komplexität der Steuerungsfunktionen für den Beschleuniger nicht derart kurzfristig mit der geforderten Präzision anpassbar, dass den Bewegungen eines Zielvolumens bzw. eines Patienten automatisch gefolgt werden kann. Vielmehr werden die Zyklen des Teilchenbeschleunigers an eine scheibenweise Abtastung des Zielvolumens angepasst.

In einer weiteren bevorzugten Ausführungsform der Erfindung weist deshalb der Tiefenmodulator zwei im Querschnitt keilförmige Ionen-Abbremsplatten auf, die das gesamte Bestrahlungsfeld des Ionenstrahls abdecken und eine schnellere Tiefenabtastanpassung bei bewegtem Zielvolumen als die Erhöhung der Ionenstrahlenergie von Energiestufe zu Energiestufe ermöglichen, sodass eine Kompensation der Eindringtiefe von Bestrahlungspunkt zu Bestrahlungspunkt mithilfe eines derartigen Tiefenmodulators möglich wird. Dazu sind die keilförmige Ionen-Abbremsplatten des Tiefenmodulators vorzugsweise auf elektromagnetisch betätigbaren Schlitten angeordnet.

Mithilfe dieser elektromagnetisch betätigbaren Schlitten kann die Position der keilförmigen Ionen-Abbremsplatten innerhalb von Bruchteilen von Millisekunden verändert werden und damit die in einem Überlappungsbereich der keilförmigen Abbremsplatten auftretende Länge des Abbremsweges der Ionen durch die Ionen-Abbremsplatten variiert werden. Dazu überlappen sich die Ionen-Abbremsplatten im Bestrahlungsbereich des Ionenstrahls und können somit die Ionen in ihrer Reichweite an örtliche und zeitliche Änderungen des bewegten Zielvolumens anpassen.

Vorzugsweise sind die Ionen-Abbremsplatten auf Linearmotoren montiert. Derartige Linearemotoren haben den Vorteil, dass eine kontinuierliche Feinregulierung der Ionenabbremsung zur Anpassung der Tiefenabtastung des Zielvolumens möglich ist. Darüber hinaus ist die Verstellung der Position der keilförmigen Ionen-Abbremsplatten mithilfe von Linearmotoren nicht nur örtlich äußerst präzise, sondern auch äußerst reaktionsschnell an eine zeitliche Verschiebung des Zielvolumens in der Tiefe anpassbar, sodass die Tiefenkompensation Bewegungen auch kontinuierlich verfolgen und kompensieren können.

Alternativ kann die Anpassung der Energie auch durch eine elektromagnetische Beschleunigungsstrecke im Beschleuniger oder in der Hochenenergiestrahlzuführung bewirkt werden.

In einer weiteren bevorzugten Ausführungsform der Erfindung weist das Positionslage- und Verfolgungssystem mindestens zwei Messaufnehmer auf, die aus zwei Raumwinkeln in Bezug auf eine Ionenstrahlachse die zeitliche und örtliche Lage von Markierungen auf einem ein Zielvolumen enthaltenen Körperbereich eines Patienten erfassen. Derartige Markierungen können mit hautverträglichen Leuchtfarben in Form von Punkten, Strichen oder anderen geometrischen Formen oder als Leuchtelemente angebracht werden, um deutlich von den Messaufnehmern wahrgenommen und vermessen zu werden.

In einer weiteren bevorzugten Ausführungsform der Erfindung sind die Messaufnehmer mindestens eine Präzisionsvideokamera und/oder ein Röntgenerfassungsmittel und/oder ein Ultraschallerfassungsmittel, die mit einer Bildauswertereinheit in dem Bewegungsmessungs-, Steuer- und Auslesemodul SAMB zusammenwirken. Damit wird vorteilhaft erreicht, dass die Bewegungen eines Körperbereichs in der Nähe eines Zielvolumens exakt vermessen werden kann und mit den zeitlichen und örtlichen Lageverschiebungen des Zielvolumens und des Abdeckgewebes beispielsweise in Form einer gespeicherten Lookup-Tabelle korreliert werden können.

Im Prinzip setzt sich die Bestrahlung eines Tumorvolumens aus Bildpunkten zusammen, die flächig in einer Scheibenform rasterförmig aneinander gesetzt werden, wobei der Ionenstrahl von Bestrahlungspunkt zu Bestrahlungspunkt orthogonal zu seiner Strahlachse in einer X- und einer Y-Richtung durch die Rasterscanvorrichtung abgelenkt wird. Wobei zur Ortserfassung die Vorrichtung eine Vieldrahtproportionalkammer als ortsempfindlichen Detektor aufweist, die strahlaufwärts von dem Tiefenmodulator angeordnet ist und Istpositionen des Ionenstrahls an ein Ortsmessungs-, Steuer- und Auslesemodul zur Kompensation von Abweichungen zwischen transversaler Istposition und transversaler Sollposition aufgrund eines Bestrahlungsplanes und aufgrund einer bewegungsbedingten Istabweichung des Zielvolumens weiterleitet.

Wenn auch die Energie der Ionen in einem Ionenstrahl durch den entsprechenden Beschleuniger konstant gehalten werden kann, so ist dennoch die Anzahl der Ionen pro Volumen über die Zeit nicht konstant. Um trotzdem eine gleich große Ionenstrahldosis in jedem Volumenpunkt des Tumorgewebes einzuhalten, und somit eine Dosis Homogenität zu schaffen, ist in einer bevorzugten Ausführungsform der Erfindung eine Ionisationskammer mit schneller Auslese zur Überwachung der Intensität des Ionenstrahlstromes als Transmissionszähler im Strahlengang des Ionenstrahls angeordnet. Ein derartiger Transmissionszähler bestimmt die Verweilzeit des Ionenstrahls auf einem zu bestrahlenden Volumenpunkt des Zielvolumens, und ein damit verbundenes Steuer- und Auslesemodul SAMI gibt ein Signal an das Auslesemodul zur Ansteuerung des nächsten Volumenpunktes ab, sobald eine vorgegebene Strahldosis erreicht ist. Somit kann in vorteilhafter Weise eine Volumenscheibe des Tumorvolumens in flächiger Erstreckung rastermäßig von Bestrahlungspunkt zu Bestrahlungspunkt abgetastet werden. Vorzugsweise ist die Ionisationskammer zwischen der Ablenkeinrichtung und dem Tiefenmodulator angeordnet, zumal der Tiefenmodulator mit seinen keilförmigen Ionen-Abbremsplatten lediglich Ionen in der Reichweite steuert, nicht aber die Ionendosis beeinflusst.

Ein Verfahren zur Kompensation von dreidimensionalen Bewegungen eines Zielvolumens auf einer Patientenliege während einer Ionenstrahl-Bestrahlung mit einer Rasterscanvorrichtung, weist die nachfolgenden Verfahrensschritte auf. Zunächst wird eine Struktur eines gesunden Gewebes, welches strahlaufwärts das Zielvolumen bedeckt in Voruntersuchungen erfasst, und ein digitales Modell der erfassten Struktur des bedeckenden gesunden Gewebes erstellt. Dieses Modell wird in einem Speicher des Bewegungs-Steuer- und Auslesemoduls SAMB beispielsweise in Form einer Lookup-Tabelle gespeichert. Auf einer Patientenliege kann dann das Zielvolumen positioniert werden.

Während der Bestrahlung werden dreidimensionale Bewegungen des Zielvolumens in Echtzeit mittels eines Positionslage- und Verfolgungssystems erfasst. Anschließend werden die Bewegungen in longitudinale und transversale Anteile vektoriell aufgeteilt, und die transversalen Anteile der Bewegungen werden durch korrigierendes Ansteuern von Rasterscanmagneten der Rasterscanvorrichtung kompensiert. Die longitudinalen Anteile der Bewegungen werden schließlich durch Abgleichen der Daten des gespeicherten Modells und durch abgleichendes Ändern der Einstellungen eines Tiefenmodulators kompensiert.

Somit wird in vorteilhafter Weise mit dieser Erfindung eine Vorrichtung zur dreidimensionalen Kompensation von Zielgebietsbewegungen in Echtzeit während der Ionenbestrahlung, beispielsweise durch Protonen oder durch schwere Ionen, bereitgestellt. Hierzu wird die transversale Strahlauslenkung durch das Rasterscansystem mit einer zusätzlichen Tiefenmodulation kombiniert. Aus der gemessenen aktuellen Abweichung der Lage und Position des jeweiligen Volumenelementes des Zielvolumens von der in einer Planung verwendeten Referenzposition, insbesondere unter Einbezug des jeweils durchstrahlten Gewebes, wird dann ein dynamischer Korrekturvektor bestimmt, und in transversale und longitudinale Komponenten zerlegt. Der longitudinalen Komponente liegt das jeweils durchstrahlte Gewebe bei der Berechnung zugrunde und sie bestimmt die benötigte Energie, um die Wechselwirkung der Ionen im jeweiligen Volumenelement im Zielvolumen zu bewirken.

Die transversalen Komponenten werden als dynamischer Offset auf die Sollposition des Rasterscansystems addiert und die longitudinale Komponente legt die Einstellung des Tiefenmodulators fest. Auf diese Wiese wird die Strahlposition in alle drei Raumrichtungen dynamisch der dreidimensionalen Zielgebietsbewegung nachgeführt. Durch eine vollständige Integration in das Bestrahlungskontrollsystem der Ionenstrahltherapieanlage mittels eines Bewegungsmessungs-, Steuer- und Auslesemodul SAMB wird der zeitliche Ablauf der Bestrahlung in der Regel nicht beeinflusst. Eine direkte Kommunikation zwischen den einzelnen Elektronikmodulen des Kontrollsystems ermöglicht die Verfügbarkeit von konsistenten, dynamischen Bewegungsdaten im gesamten System.

Dazu wird vorzugsweise im Vorfeld der Bestrahlung ein digitales Strukturmodell des Gewebes, welches das Zielvolumen strahlaufwärts bedeckt, durch Röntgen- und/oder Ultraschalluntersuchungen erfasst. Mit derartigen Untersuchungen im Vorfeld der Bestrahlung lässt sich sehr präzise die Tiefenänderung der Dosiseskalation bei der Ionenbestrahlung bei einer Bewegung in Echtzeit unter Nachführen des Tiefenmodulators kompensieren, in dem die longitudinale Tiefenkorrektur über den Tiefenmodulator von Strahlposition zu Strahlposition durchgeführt wird.

In einem bevorzugten Durchführungsbeispiel des Verfahrens wird die Ortsmessung mittels einer Vieldrahtproportionalkammer über ein Ortsmessungs-, Steuer- und Auslesemodul SAMO registriert und ausgewertet. Zur transversalen Kompensation wird eine in dem Ortsmessungs-, Steuer- und Auslesemodul SA-MO eines Kontrollsystems abgelegte Information über die Sollposition einer Bestrahlungsplanung mit der gemessenen Istposition der Strahlposition aus dem ortsempfindlichen Detektor in Echtzeit unter Berücksichtigung der erfassten transversalen Bewegungskomponente des Zielvolumens verglichen und die Ortskompensation transversal in X- und Y-Richtung über die schnellen Scannermagnet-Netzgeräte in Zusammenwirken mit einem Steuer- und Auslesemodul SAMS für die Rasterscanmagnete der Rasterscanvorrichtung durchgeführt.

Eine kontrollierte, kurzzeitige Unterbrechung beim Auftreten von unvorhergesehenen Bewegungszuständen außerhalb des Arbeitsbereiches der Kompensation gewährleistet in einem bevorzugten Durchführungsbeispiel des Verfahrens, den flexiblen und dennoch sicheren Einsatz für jegliche Art von Bewegungen. Dazu wird eine schnelle Strahlabschaltung von dem Ortsmessungs-, Steuer- und Auslesemodul SAMO des ortsempfindlichen Detektors in Echtzeit und/oder dem Bewegungs-, Steuer- und Auslesemodul SAMB des Tiefenmodulators ausgelöst, falls die Differenz zwischen einem Messwert und einem Sollwert der transversalen Strahlposition bzw. der longitudinalen Eindringtiefe, einen in der Echtzeitsoftware der Steuer- und Auslesemodule SAMO und/oder SAMB einstellbaren Schwellenwert übersteigt.

Außerdem übernimmt das SAMB neben der Korrektur der Sollposition des Ionenstrahls und der Ansteuerung des Tiefenmodulators die Überwachung der angeschlossenen Subsysteme für eine Positionslage- und Lageverfolgungsmethode und zur Tiefenmodulation gegen Fehler und Ausfall. Das SAMB überprüft die resultierenden Werte auf Konsistenz und Schlüssigkeit und löst im Fehlerfall ein entsprechendes Interlocksignal, welches die Bestrahlung abbricht, aus. Überschreiten die notwendigen Korrekturparameter die im Vorfeld festgelegte Limits, so wird die Bestrahlung kurzzeitig unterbrochen, bis die Werte wieder innerhalb des erlaubten Bereichs sind.

Die Erfindung wird nun anhand der beigefügten Figuren 3-6 näher erläutert.
- Figur 3: zeigt eine Prinzipskizze eines Anordnungs- und Ver- bindungsschemas der Komponenten einer Vorrichtung, gemäß einer Ausführungsform der Erfindung.
- Figur 4: zeigt ein schematisches Blockschaltbild der Steuer- und Auslesemodule mit angeschlossenen externen Gerä- ten, einer Vorrichtung gemäß Figur 3.
- Figur 5: zeigt schematische Ergebnisse einer Bewegungskompen- sation in Bezug auf eine relative Dosishomogenität in Abhängigkeit von der Eindringtiefe, wenn die Be- wegung mit unterschiedlicher Varianz von 0,0 mm bis 3,0 mm erfasst werden kann.
- Figur 6: zeigt schematisch Ergebnisse einer Bewegungskompen- sation in vergrößertem Maßstab in Bezug auf eine prozentuale Abweichung von einer relativen Dosisho- mogenität in Abhängigkeit von der Eindringtiefe bei statischem Zielvolumen, wenn die Bewegung mit einer Varianz von 0,5 mm, 1,0 oder 1,5 mm erfasst werden kann.
- Figur 7: zeigt schematisch ein Blockdiagramm einer Vorrich- tung gemäß einer Ausführungsform der Erfindung.
- Figur 8: zeigt schematisch einen statischen Fall der Bestrah- lung eines Zielvolumens.
- Figur 9: zeigt schematisch einen dynamischen Fall der Be- strahlung eines statischen Zielvolumens mit einem sich bewegenden Deckvolumen.
- Figur 10: zeigt schematisch einen dynamischen Fall der Be- strahlung eines sich bewegenden Zielvolumens und ei- nes sich bewegenden Deckvolumens.

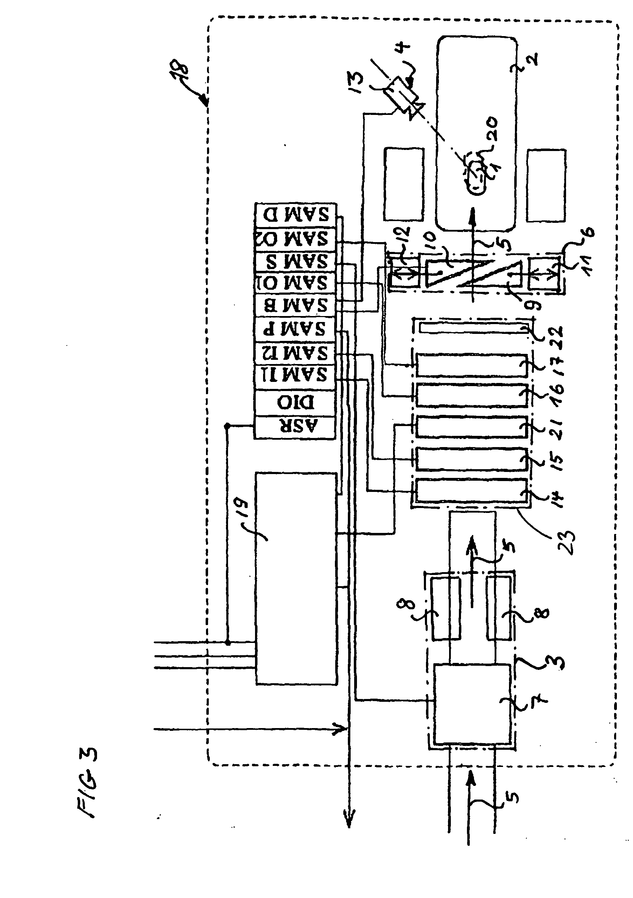

Figur 3 zeigt eine Prinzipskizze eines Anordnungs- und Verbindungsschemas der Komponenten einer Vorrichtung, gemäß einer ersten Ausführungsform der Erfindung. Dazu kennzeichnet das Bezugszeichen 1, ein Zielvolumen und die gestrichelte Linie 20 deutet eine dreidimensionale Bewegung des Zielvolumens 1 auf einer Patientenliege 2 an. Das Bezugszeichen 3 kennzeichnet eine Rasterscanvorrichtung, die einen Rasterscanmagneten 7 für eine X-Auslenkung eines Ionenstrahls 5 aus Protonen oder schweren Ionen aufweist, und die einen weiteren Rasterscanmagneten 8 für eine Y-Ablenkung des Ionenstrahls 5 aufweist.

Der Ionenstrahl 5 passiert nach der Rasterscanvorrichtung 3 mehrere Messkammern 23. Davon dienen Ionisationskammern 14 und 15 der Erfassung der Dosis des Ionenstrahls 5 und Vieldrahtproportionalkammern 16 und 17 der Messung der Ortspositionen des Ionenstrahls 5 und sind dazu im Ionenstrahl 5 strahlaufwärts eines Tiefenmodulators 6 angeordnet. Ferner passiert der Ionenstrahl 5 eine Zusatzmesskammer 21 zur Grenzwertüberwachung, die unmittelbar mit einem Kontrollrechner 19 des Bestrahlungsraums 18 in Wirkverbindung steht. Darüber hinaus passiert der Ionenstrahl 5 noch vor dem Tiefenmodulator 6 ein Kammfilter 22.

Der Tiefenmodulator 6 ist vor dem Zielvolumen 1 angeordnet, und weist zwei keilförmige Ionenbremsplatten 9 und 10 auf, die zur Tiefenmodulation mit Hilfe von Linearmotoren 11 und 12 gegeneinander verstellt werden können, um die Eindringtiefenkompensation unter Berücksichtigung der Änderungen des gesunden Gewebes und der Lageänderung des Zielvolumens 1 bei Bewegungen des Patienten auf der Patientenliege 2 durchzuführen.

Zur Erfassung der Bewegung weist der Bestrahlungsraum 18 ein Positionslage- und Verfolgungssystem 4 auf, das als Messaufnehmer mindestens eine Präzisionsvideokamera 13 und/oder ein Röntgenerfassungsmittel und/oder ein Ultraschallerfassungsmittel aufweist, die mit einem Steuer- und Auslesemodul SAMB für eine Bewegungskompensation in Wirkverbindung steht.

Figur 3 zeigt somit, eine Ausführungsform eines erfindungsgemäßen Bestrahlungssystems für ein intensitäts-, orts- und bewegungsmoduliertes Rasterscanverfahren. Dieser Hardwareüberblick der Figur 3 stellt eine Weiterentwicklung des Systems der in DE 100 31 074 A1 beschriebenen Gegenstände dar, wodurch die Präzisionsbestrahlung von bewegten Zielgebieten verbessert wird. Die Verbesserung in diesem Bestrahlungssystem wird durch die Zusammenführung von Rasterscansystem und Tiefenmodulator erreicht, sowie durch die Verarbeitung einer in Echtzeit gemessenen Bewegungsinformation durch Hinzufügen eines weiteren Elektronikmoduls SAMB zum Kontrollsystem und durch Verwendung weiterer Kommunikationsschnittstellen und verbesserter digitaler Modelle.

Die Erfindung ermöglicht somit ein verbessertes, dynamisches, dreidimensionales Nachführen des Therapiestrahls in Echtzeit mit feiner Auflösung und extrem hoher Genauigkeit.

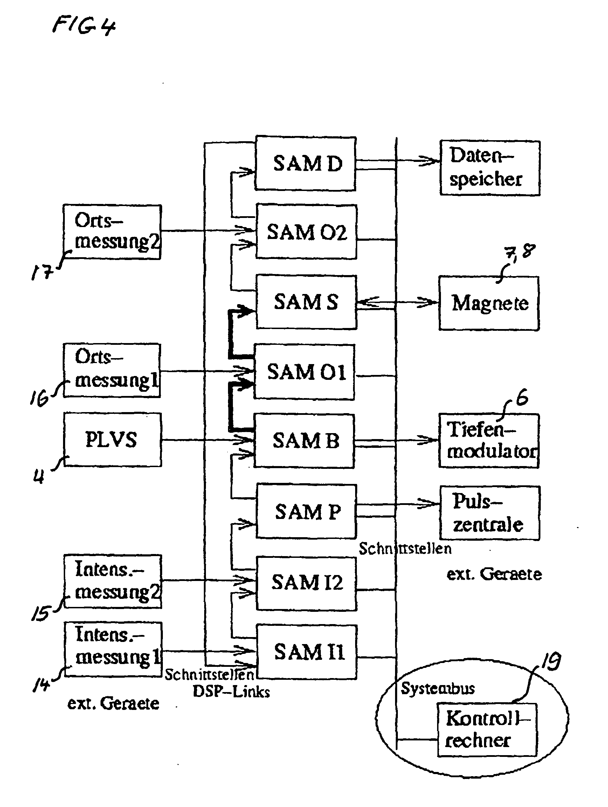

Figur 4 zeigt ein schematisches Blockschaltbild der Steuer- und Auslesemodule SAM mit angeschlossenen externen Geräten, einer Vorrichtung gemäß Figur 3. Mit Figur 4 wird gezeigt, dass die erfindungsgemäße Vorrichtung voll in ein Bestrahlungskontrollsystem integriert ist, welches sich aus einer System- und einer Ablaufsteuerung und einem Kontrollrechner 19 zusammensetzt. In diesem Ausführungsbeispiel besteht die Ablaufsteuerung des Kontrollsystems aus mehreren Elektronikmodulen, den Steuerungs- und Auslesemodulen SAM mit verschiedenen Aufgaben, sowie dem Ablaufsteuerungsrechner ASR.

Ferner existiert ein eigenes Modul SAMB ausschließlich für die Kompensation von Zielgebietsbewegungen. Unter sicherheitstechnischen Gesichtspunkten kann ein zweites, identisches Modul verwendet werden, welches Konsistenzprüfungen des Datenstromes zulässt. Das SAMB-Modul befindet sich in der Datenkette, die in Figur 4 durch Pfeile gekennzeichnet wird, beispielsweise vor der Ortsmessung SAMO 1. Die Echtzeitsoftware auf dem SAMB liest in einem festen Zeitintervall die Bewegungsinformation aus dem über eine Schnittstelle angeschlossenen Positions- und Lageverfolgungssystem 4, und bestimmt unter Zuhilfenahme einer im Vorfeld berechneten Lookup-Tabelle den erforderlichen Kompensationsvektor im Bezugssystem der Bestrahlungsbehandlung.

Die Frequenz der Bewegungsmessung kann frei an die jeweilige Positionslage- und Lageverfolgungsmethode, sowie an die erforderliche Messgenauigkeit mit beispielsweise 10 Hz bis 100 Hz angepasst werden. Überschreitet die Länge des Messintervalls die Dauer der Ortsmessung im Kontrollsystem, so bleibt der ermittelte Kompensationsvektor bis zum nächsten Zyklus der Bewegungserfassung mit Hilfe des Positionslage- und Lageverfolgungssystems 4 aktuell.

In jedem Messzyklus der schnellen Ortskorrektur, in beispielsweise 150 µs, ermittelt das SAMB die aktuelle transversale Strahlposition aus dem gespeicherten Solldatensatz und versieht sie mit den transversalen Komponenten des aktuellen Kompensationsvektors. Diese neue Sollposition wird über eine Schnittstelle in der Echtzeitsteuerung über SAMO 1 an die Ansteuerung SAMS der Rasterscanmagnete 7 und 8 weitergegeben, welche diesen Wert mit der aktuellen gemessenen Istposition von SAMO 1 vergleicht und gegebenenfalls über eine Regelschleife die transversale Strahlposition korrigiert.

Die modifizierte Sollposition und die Bewegungsinformation werden zu Zwecken der Protokollierung und Datenkonsistenz an alle anderen Module des Kontrollsystems weitergegeben. Weiterhin berechnet SAMB aus der longitudinalen Strahl-Sollposition und der ermittelten longitudinalen Kompensationskomponente die Einstellungen des Tiefenmodulators 6. Die Ansteuerung des Tiefenmodulators 6 erfolgt direkt über eine Schnittstelle des SAMB. Mit Figur 4 werden somit exemplarisch der Datenfluss und die erforderlichen Schnittstellen gezeigt.

Figur 5 zeigt schematische Ergebnisse einer Bewegungskompensation mit den Kurven g bis n in Bezug auf eine relative Dosishomogenität DH in Abhängigkeit von der Eindringtiefe w in mm, wenn die Bewegungskompensation mit unterschiedlicher Varianz von σ = ± 0,0 mm (Kurve g) bis σ = ± 3,0 mm (Kurve n) durchgeführt werden kann. Durch die Kompensation mit Hilfe der erfindungsgemäßen Vorrichtung wird somit eine Dosishomogenität erreicht, die trotz bewegtem Zielvolumens annähernd der Dosishomogenität bei statischem, d.h. auf der Patientenliege fixiertem Zielvolumen, erreicht wird. Diesen Ergebnissen wird mit der Kurve f die relative Dosishomogenität ohne Kompensationsmaßnahmen, wie sie auch in Figur 2 gezeigt wird, vergleichsweise gegenübergestellt.

Figur 6 zeigt schematisch Ergebnisse einer Bewegungskompensation in vergrößertem Maßstab in Bezug auf eine prozentuale Abweichung ΔDH der Dosishomogenität in Prozent von der Dosishomogenität DH eines statischen Zielvolumen, in Abhängigkeit von der Eindringtiefe w in mm, wenn die Bewegungen mit einer Varianz von σ = ± 0,5 mm (Kurve i), σ = 1,0 mm (Kurve k) und σ = 1,5 mm (Kurve 1) erfasst und kompensiert werden kann.

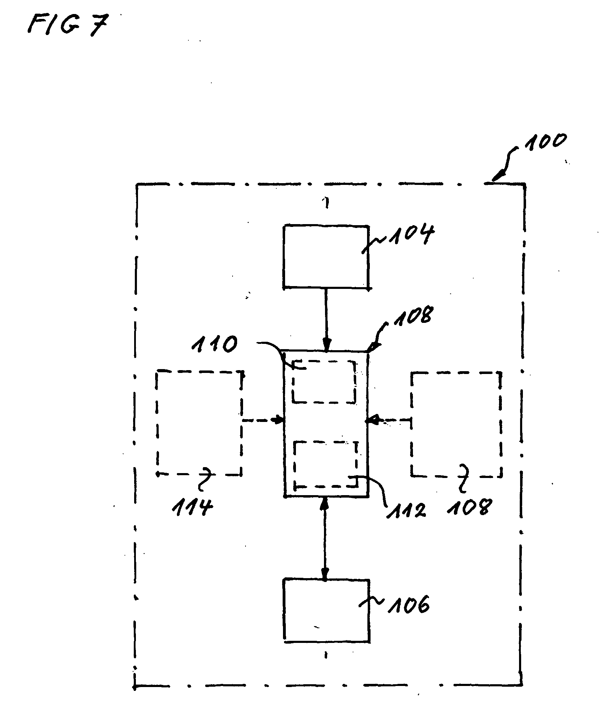

Figur 7 erläutert die Erfindung beispielhaft an einer Vorrichtung 100 zur Anpassung der in den Figuren 1, 8, 9 und 10 gezeigten Eindringtiefe W eines Ionenstrahls in Abhängigkeit von einer Bewegung eines Patienten, d.h. in Abhängigkeit von der Bewegung von Körperregionen des Patienten, z.B. der A-tembewegung des Brustkorbes. Die Vorrichtung 100 umfasst ein Positionslage- und Verfolgungssystem 104 zur Überwachung von Bewegungen des Patienten, einen Tiefenmodulator 106 zur Einstellung der Eindringtiefe des Ionenstrahls in den Patienten und eine Bewegungsmessungs- und Steuereinheit 108, die mit dem Positionslage- und Verfolgungssystem 104 und dem Tiefenmodulator 106 wirkverbunden ist.

Die Bewegungsmessungs- und Steuereinheit 108 umfasst einen Mikroprozessor 110 mit einem Speicher, auf dem Daten eines Modells 112 abgelegt sind. Das Modell 112 beschreibt die Struktur von gesundem Gewebe, welches strahlaufwärts das Zielvolumen abdeckt und somit vom Ionenstrahl bei der Bestrahlung durchstrahlt werden muss. Ein derartiges Modell ist beispielsweise aus "A.Schweikard et al: Robotic motion compensation for respiratory movement during radiosurgery. Comput Aided Surg. 2000;5(4):263-77" bekannt.

Die Bewegungsmessungs- und Steuereinheit 108 erhält Informationen über die Bewegung des Patienten vom Positionslage- und Verfolgungssystem 104. Sie verarbeitet mit Hilfe des Mikroprozessors diese Information zusammen mit dem Modell, um ein Steuersignal für den Tiefenmodulator 106 bereitzustellen. Dieses Steuersignal soll den Tiefenmodulator 106 derart ansteuern, dass die Eindringtiefe des Ionenstrahls unabhängig von der Bewegung des Patienten jeweils auf das gerade zu bestrahlende Zielvolumenelement des Zielvolumens (Tumors) im Patienten eingestellt ist. (Im Folgenden wird Zielvolumen und Zielvolumenelement z.T. als gleichbedeutend benutzt, da sich die genauere Bedeutung jeweils aus dem Zusammenhang ergibt.) Dazu wird insbesondere die Bewegung des gesunden Gewebes relativ zum Zielvolumenelement benötigt, da in Abhängigkeit vom durchstrahlten gesunden Gewebe unterschiedliche Energieabsorptionen des Teilchenstrahls im Patienten stattfinden und sich somit die Eindringtiefe des Ionenstrahls in Abhängigkeit vom durchstrahlten Gewebe bei einer relativen Bewegung vom gesunden Gewebe zum Zielvolumenelement ändert. Die Reichweitenänderung des Ionenstrahls in Abhängigkeit vom durchstrahlten Gewebe kann beispielsweise online während der Bestrahlung berechnet oder mithilfe von z.B. bei der Therapieplanung erzeugten Tabellen, die verschiedene Gewebeanordnungen repräsentieren, bestimmt werden.

In einer erweiterten Ausführungsform der Vorrichtung 100 zur Anpassung der Eindringtiefe kann dieses zusätzliche Mittel 114 zur Gewinnung von Lageinformationen über die Lage des Ionenstrahls relativ zum Patienten aufweisen. Diese Lageinformation kann wiederum zusammen mit Hilfe des Modells und der Information über die Bewegung des Patienten zur Ansteuerung einer Rasterscanvorrichtung 116 genutzt werden. Dadurch kann der Ionenstrahl einer Bewegung des Zielvolumens transversal zum Ionenstrahl folgen, wobei gleichzeitig in Abhängigkeit von der relativen Bewegung des gesunden Gewebes zum Zielvolumen die Eindringtiefe angepasst werden kann.

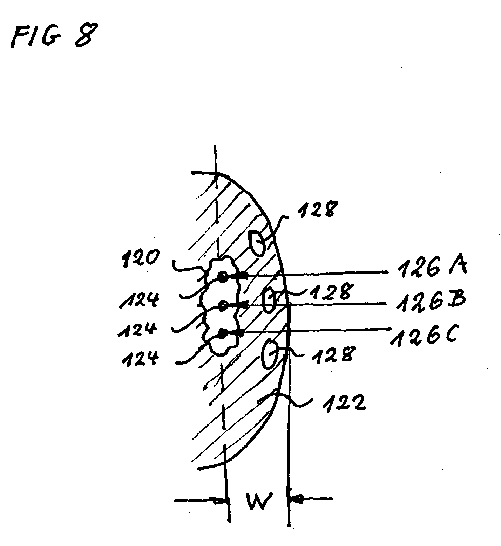

Figur 8 zeigt einen erfindungsrelevanten Aspekt bei der Strahlentherapie in einem statischen Fall, bei dem sich weder das Zielvolumen 120 bzw. Tumorgewebe 120 noch das bei der Therapie durchstrahlte Deckgewebe 122 bzw. gesunde Gewebe 122 bewegen. Schematisch werden 3 Bestrahlungspunkte 124 (Zielvolumenelemente) gezeigt, die jeweils mit den Ionenstrahlen 126A, 126B und 126C bestrahlt werden. Bei der Therapieplanung wird das durchstrahlte Gewebe 122 analysiert. Zum Erreichen der Strahlposition 124 in einer Tiefe W wird die Ionenstrahlenergie auf das durchstrahlte Deckvolumen 122 angepasst. Dabei wird berücksichtigt, ob beispielsweise Knochen 128 durchstrahlt werden, wie es für den Ionenstrahl 126B der Fall ist, oder nicht (Ionenstrahlen 126A und 126C).

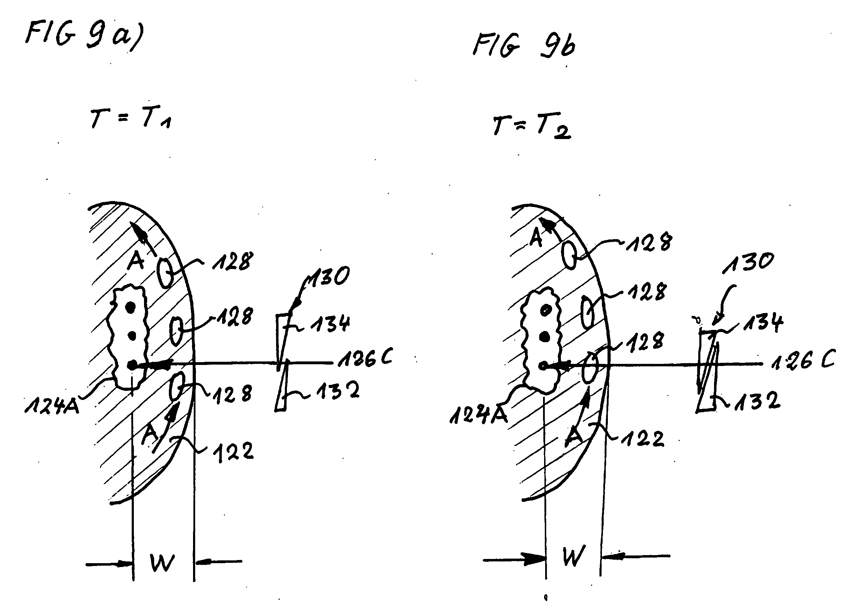

Figur 9 erweitert das Schema aus Figur 8 nun um eine Bewegung des Patienten, wobei hier beispielsweise die Knochen 128 den Rippen des Brustkorbes entsprechen, die sich bei der Atmung in Pfeilrichtung A relativ zu dem statischen Zielvolumen 120 verschieben. Im Falle eines nicht bewegten Tumorgewebes 124A ändert sich jetzt für die Strahlposition 126C während der Atmung und während der Bestrahlung die Zusammensetzung des jeweils durchstrahlten Deckvolumens 122: Zum Zeitpunkt T=T1 wird keine Rippe 128 und zum Zeitpunkt T=T2 wird eine Rippe 128 durchstrahlt. Entsprechend muss die Strahlenenergie zeitabhängig angepasst werden, um bei der Bestrahlung die Eindringtiefe W des Ionenstrahls 126C zu gewährleisten. Dazu überwacht das Positionslage- und Verfolgungssystem Bewegungen des Patienten, hier die Bewegungen des Brustkorbs, und übermittelt diese Information an die Bewegungsmessungs- und Steuereinheit in der das Modell des durchstrahlten Deckvolumens 122 abgeglichen wird und eine entsprechende Tiefenmodulation eines Tiefenmodulators 130 veranlasst. Beispielsweise überlappen sich die Keile 132 und 134 des Tiefenmodulators 130 zum Zeitpunkt T2 mehr als zum Zeitpunkt T1.

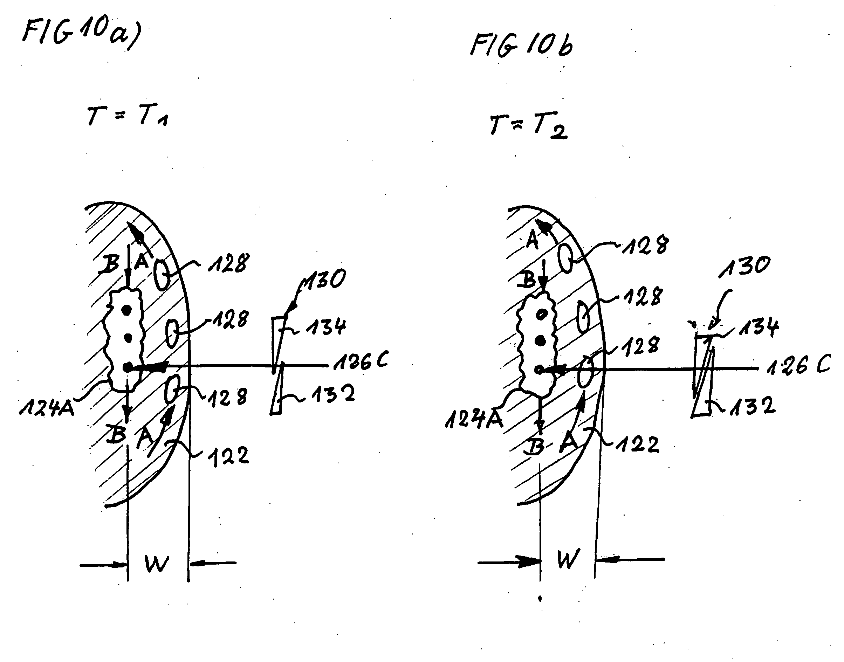

Figur 10 berücksichtigt nun zusätzlich die Bewegung des Zielvolumens 124B Richtung B selbst. Die Überwachung von Bewegungen des Patienten lassen dabei über das Modell auch eine Bestimmung der Bewegung des Zielvolumens zu. Beispielsweise hat sich in Figur 10b das Tumorgewebe 124B zum Zeitpunkt T2 nach unten in Pfeilrichtung B und das gesunden Gewebe 122 nach oben in Pfeilrichtung A bewegt. Die Aufgabe einer Strahlnachführungsvorrichtung, beispielsweise einer Rasterscanvorrichtung, ist es, den Ionenstrahl 126C dem beispielsweise 126C parallel nach unten zu verschieben, so dass immer der jeweilige Bestrahlungspunkt 124 bestrahlt wird. Erfindungsgemäß ist es nun auch möglich, mit Hilfe des Modells die geänderten Bedingungen aufgrund der Relativbewegung von Tumorgewebe 124B und gesundem Gewebe 122 bei der Durchstrahlung des gesunden Gewebes 122 zu berücksichtigen und entsprechend wiederum den Tiefenmodulator anzusteuern.

Die Möglichkeiten, die Korrektur der Eindringtiefe erfindungsgemäß durchzuführen, beschränkt sich nicht nur auf die bekannte und eingangs skizzierte Rasterscanmethode bei der Partikeltherapie, sondern sie kann auch bei der intensitätsmodulierten Bestrahlung verwendet werden. Dabei werden im Gegensatz zur Rasterscanmethode kleine Feldbereiche z.B. mit Multileaf-Kollimatoren ausgeblendet und mit verschiedenen Intensitäten aneinandergesetzt.

### Bezugszeichenliste

- 1: Zielvolumen
- 2: Patientenliege
- 3: Rasterscanvorrichtung
- 4: Positionslage- und Verfolgungssystem
- 5: Ionenstrahl
- 6: Tiefenmodulator
- 7: Rasterscanmagnet (X-Ablenkung)
- 8: Rasterscanmagnet (Y-Ablenkung)
- 9: keilförmige Ionenbremsplatte
- 10: keilförmige Ionenbremsplatte
- 11: Linearmotor
- 12: Linearmotor
- 13: Präzisionsvideokamera; Röntgenerfassungsmittel; Ultra- schallerfassungsmittel
- 14: Ionisationskammer
- 15: Ionisationskammer
- 16: Vieldrahtproportionalkammer
- 17: Vieldrahtproportionalkammer
- 18: Behandlungsraum
- 19: Kontrollrechner
- 20: gestrichelte Linie
- 21: Zusatzmesskammer
- 22: Kammfilter
- 23: Messkammern

- 100: Vorrichtung zur Anpassung der Eindringtiefe
- 104: Positionslage- und Verfolgungssystem
- 106: Tiefenmodulator
- 108: Bewegungs- und Steuereinheit
- 110: Mikroprozessor
- 112: Modell
- 120: Zielvolumen bzw. Tumorgewebe
- 122: durchstrahltes Deckvolumen bzw. gesundes Gewebe
- 124: Bestrahlungspunkt
- 124A: Strahlposition
- 126A: Ionenstrahl
- 126B: Ionenstrahl
- 126C: Ionenstrahl
- 128: Knochen
- 130: Tiefenmodulator
- 132: Keil
- 134: Keil

- A: Pfeilrichtung
- B: Pfeilrichtung
- T: Zeit
- T₁: Zeitpunkt
- T₂: Zeitpunkt Differenz zwischen Messwert und Sollwert
- Δw: Änderung der Eindringtiefe w Eindringtiefe

- SAMO1: Ortsmessungs-, Steuer- und Auslesemodul
- SAMO2: Ortsmessungs-, Steuer- und Auslesemodul
- SAMS: Steuer- und Auslesemodul der Rasterscanmagnete
- SAMB: Bewegungsmessungs-, Steuer- und Auslesemodul

## Patentansprüche

1. Vorrichtung zur Kompensation von dreidimensionalen Bewegungen eines Zielvolumens (1) auf einer Patientenlagcrungsvorrichtung (2) während einer Ionenstrahl-Bestrahlung mit einer Rasterscanvorrichtung (3), wobei die Kompensationsvorrichtung umfasst:
- ein Positionslage- und Verfolgungssystem (4), das die dreidimensionalen Bewegungen des Zielvolumens (1) erfasst, und
- einen Tiefenmodulator (6), der die Eindringtiefe w des Ionenstrahls (5) nachregelt,
- die Rasterscanvorrichtung (3), die den Ionenstrahl (5) transversal ablenkt, und mit einem Ortsmessungs-, Steuer- und Auslesemodul (SAMO) und einem Modul zur Änderung der Strahlauslenkung (SAMS) in Wirkverbindung steht,
**dadurch gekennzeichnet, dass**
das Positionslage- und Verfolgungssystem (4) mit einem Bewegungsmessungs-, Steuer- und Auslesemodul SAMB in Wirkverbindung steht, und dass der Tiefenmodulator (6) mit dem Bewegungsmessungs-, Steuer- und Auslesemodul SAMB in Wirkverbindung steht, und wobei das Bewegungsmessungs-, Steuer- und Auslesemodul (SAMB) einen Mikroprozessor mit Speicher aufweist, und wobei der Speicher Daten eines Modells einer Struktur eines gesunden Gewebes, das strahlaufwärts das Zielvolumen (1) bedeckt, aufweist, und wobei der Mikroprozessor Rechenkomponenten aufweist, welche die erfassten Bewegungen des Zielvolumens (1) vektoriell in longitudinale und transversale Anteile zum Ionenstrahl (5) gliedern und welche die longitudinalen Anteile mit dem gespeicherten Modell zur Korrektur der Eindringtiefe w des Ionenstrahls(5) abgleichen.

2. Vorrichtung nach Anspruch 1, **dadurch gekennzeichnet, dass** die Rasterscanvorrichtung (3) zwei Rasterscanmagnete (7, 8) aufweist, die einen Ionenstrahl (5) orthogonal in Bezug auf eine Einkopplungsrichtung in die Rasterscanmagnete (7, 8) in zwei Richtungen, die ihrerseits senkrecht zueinander liegen, zum scheibenweisen Flächenabtasten des Zielvolumens (1) ablenken.

3. Vorrichtung nach Anspruch 2, **dadurch gekennzeichnet, dass** die Rasterscanmagnete (7, 8)durch reaktionsschnelle Netzgeräte gesteuert werden.

4. Vorrichtung nach einem der vorhergehenden Ansprüche, **dadurch gekennzeichnet, dass** die Vorrichtung Ionen-Beschleunigungselemente aufweist, mit denen die Energie des Ionenstrahls (5) einstellbar ist, sodass das Zielvolumen (1) scheibenweise in der Eindringtiefe w gestaffelt bestrahlbar ist.

5. Vorrichtung nach einem der vorhergehenden Ansprüche, **dadurch gekennzeichnet, dass** der Tiefenmodulator (6) zwei im Querschnitt keilförmige Ionen-Abbremsplatten (9, 10) aufweist, die das gesamte Bestrahlungsfeld des gescannten Ionenstrahls (5) abdecken.

6. Vorrichtung nach Anspruch 5, **dadurch gekennzeichnet, dass** die Ionen-Abbremsplatten (9, 10) auf Linearmotoren (11, 12) montiert sind.

7. Vorrichtung nach Anspruch 5, **dadurch gekennzeichnet, dass** die Ionen-Abbremsplatten (9, 10) auf elektromagnetisch betätigbaren Schlitten angeordnet sind.

8. Vorrichtung nach einem der Ansprüche 5 bis 7, **dadurch gekennzeichnet, dass** die Ionen-Abbremsplatten (9, 10) mit ihrem keilförmigen Querschnitt überlappend im Bereich des Ionenstrahls (5) gegeneinander verschiebbar sind.

9. Vorrichtung nach einem der Ansprüche 1 bis 8, **dadurch gekennzeichnet, dass** das Positionslage- und Verfolgungssystem (4) mindestens eine Präzisionsvideokamera (13) und/oder ein Röntgenstrahlerfassungsmittel und/oder ein Ultraschallerfassungsmittel aufweist, die mit einer Bildauswerteeinheit in dem Bewegungsmessungs-, Steuer- und Auslesemodul SAMB in Wirkverbindung stehen.

10. Vorrichtung nach einem der vorhergehenden Ansprüche, **dadurch gekennzeichnet, dass** eine Ionisationskammer in (14, 15) mit schneller Auslese zur Überwachung der Intensität des Ionenstrahlstroms als Transmissionszähler im Strahlengang des Ionenstrahls (5) angeordnet ist.

11. Vorrichtung nach Anspruch 10, **dadurch gekennzeichnet, dass** die Ionisationskammer (14, 15) zwischen der Rasterscanvorrichtung (3) und dem Tiefenmodulator (6) angeordnet ist.

12. Vorrichtung nach Anspruch 11, **dadurch gekennzeichnet, dass** eine Vieldrahtproportional kammer (16, 17) als ortsempfindlicher Detektor strahlaufwärts von dem Tiefenmodulator (6) angeordnet ist.

13. Vorrichtung zur Anpassung der Eindringtiefe eines Ionenstrahls in Abhängigkeit von einer Bewegung eines Patienten auf einer Patientenlagerungsvorrichtung einer Therapieanlaqe, umfassend
- ein Positionslage- und Vorfolgungssystem (4) zur Überwachung von Bewegungen des Patienten,
- einen Tiefenmodulator zur Einstellung der Eindringtiefe des Ionenstrahls in den Patienten und
- eine Bewegungsmessungs- und Steuereinheit, die mit dem Positionslage- und Verfolgungssystem (4) und dem Tiefen modulator verbunden ist und vom Posilionslage- und Verfolgungssystem (4) Information über die Bewegung des Pa tienten erhält sowie den Tiefenmodulator zur Anpassung der Eindringtiefe ansteuert,
**dadurch gekennzeichnet, dass** die Bewegungsmessungs- und Steuereinheit einen Mikroprozessor mit einem Speicher aufweist, und wobei der Speicher Daten eines Modells einer Struktur von gesundem Gewebe, das strahlaufwärts das Zielvolumen abdeckt, aufweist und wobei der Mikroprozessor mithilfe des Modells und der Information über die Bewegung des Patienten den Tiefenmodulator derart ansteuert, dass die Eindringtiefe des Ionenstrahls unabhängig von der Bewegung des Patienten, insbesondere unabhängig von der Bewegung des gesunden Gewebes relativ zum Zielvolumen, auf ein Zielvolumenelement im Patienten eingestellt ist.

14. Vorrichtung nach Anspruch 1.3, **dadurch gekennzeichnet, dass** aus dem Modell die Energieabsorption des durchstrahlten Gewebes und damit die Reichweitenänderung des Ionenstrahls in Abhängigkeit vom durchstrahlten Gewebe berechenbar ist.

15. Vorrichtung nach Anspruch 13 oder 14, **dadurch gekennzeichnet, dass** aus der Information über die Bewegung des Patienten und dem Modell das zu durchstrahlende Gewebe bestimmbar isL.

16. Vorrichtung nach einem der Ansprüche 13 bis 15, **dadurch gekennzeichnet, dass** das Modell Änderungen in der Elektronendichteverteilung im gesunden Gewebe, insbesondere über mehrdimensionale Röntgenprojektionsaufnahmen oder aus zeitaufgelösten CT-Datensätzen gewonnen, mit Bewegungszuständen des Körpers korreliert.

17. Vorrichtung nach einem der Ansprüche 13 bis 16, **dadurch gekennzeichnet, dass** der Tiefenmodulator zur Änderung der Eindringtiefe eine Vorrichtung zur Änderung der kinetischen Energie der Ionen aufweist.

18. Vorrichtung nach einem der Ansprüche 13 bis 17, **dadurch gekennzeichnet, dass** die Vorrichtung zusätzlich Mittel zur Gewinnung von Lageinformation über die Lage des Ionenstrahls relativ zum Patienten aufweist, wobei die Bewegungsmessungs- und Steuereinheit aus der Lageinformation zusammen mithilfe des Modells und der Information über die Bewegung des Patienten eine Rastorscanvorrichtung derart ansteuert, dass der Ionenstrahl einer Bewegung des Zielvolumens transversal zum Ionenstrahl folgt.

19. Vorrichtung nach einem der Ansprüche 13 bis 18, **dadurch gekennzeichnet, dass** die Rasterscanvorrichtung (3) zwei Rasterscanmagnete (7, 8) aufweist, die einen Ionenstrahl (5) orthogonal in Bezug auf eine Einkopplungsrichtung in die Rasterscanmagnete (7, 8) in zwei Richtungen, die ihrerseits senkrecht zueinander liegen, zum scheibenweisen Flächenabtasten des Zielvolumens (1) ablenken.

20. Vorrichtung nach Anspruch 19, **dadurch gekennzeichnet, dass** die Rasterscanmagnete (7, 8) durch reaktionsschnelle Netzgeräte gesteuert werden.

21. Vorrichtung nach einem der Ansprüche 13 bis 20, **dadurch gekennzeichnet, dass** die Vorrichtung Ionen-Beschleunigungselemente aufweist, mit denen die Energie des Ionenstrahls (5) einstellbar ist, sodass das Zielvolumen (1) scheibenweise in der Eindringtiefe w gestaffelt bestrahlbar ist.

22. Vorrichtung nach einem der Ansprüche 13 bis 21, **dadurch gekennzeichnet, dass** der Tiefenmodulator (6) zwei im Querschnitt keilförmige ionen-Abbremsplatten (9, 10) aufweist, die das gesamte Bestrahlungsfeld des gescannten Ionenstrahls (5) abdecken.

23. Vorrichtung nach Anspruch 22, **dadurch gekennzeichnet, dass** die Ionen-Abbremsplatten (9, 10) auf Linearmotoren (11, 12) montiert sind.

24. Vorrichtung nach Anspruch 22, **dadurch gekennzeichnet, dass** die Ionen-Abbremsplatten (9, 1.0) auf elektromagnetisch betätigbaren Schlitten angeordnet sind.

25. Vorrichtung nach einem der Ansprüche 22 bis 24, **dadurch gekennzeichnet, dass** die Ionen-Abbremsplatten (9, 10) mit ihrem keilförmigen Querschnitt überlappend im Bereich des Ionenstrahls (5) gegeneinander verschiebbar sind.

26. Vorrichtung nach einem der Ansprüche 13 bis 25, **dadurch gekennzeichnet, dass** das Positionslage- und Verfolgungssystem (4) mindestens eine Präzisionsvideokamera (13) und/oder ein Röntgenstrahlerfassungsmittel und/oder ein Ultraschallerfassungsmittel aufweist, die mit einer Bildauswerteeinheit in dem Bewegungsmessungs-, Steuer- und Auslesemodul SAMB in Wirkverbindung stehen.

27. Vorrichtung nach einem der Ansprüche 13 bis 26, **dadurch gekennzeichnet, dass** eine Vieldrahtproportionalkammer (16, 17) als ortsempfindlicher Detektor strahlaufwärts von dem Tiefenmodulator (6) angeordnet ist.

28. Vorrichtung nach einem der Ansprüche 13 bis 27, **dadurch gekennzeichnet, dass** die Vorrichtung zum Erfassen der Struktur des gesunden Gewebes, welches das Zielvolumen strahlaufwärts bedeckt, ein Röntgenstrahl- und/oder ein Ultraschallerfassungsgerät zur Verwendung im Vorfeld und während einer Ionenstrahl-Bestrahlung aufweist.

29. Vorrichtung nach einem der Ansprüche 13 bis 28, **dadurch gekennzeichnet, dass** die Rasterscanmagnete (7, 8) Scannermagnetstrom-Netzgeräte für eine horizontale und eine vertikale Korrektur über Steuer- und Auslesemodule (SAMS) für die Rasterscanmagnete (7, 8) aufweisen.

30. Vorrichtung nach einem der Ansprüche 13 bis 29, **dadurch gekennzeichnet, dass** eine Vieldrahtproportionalkammer (16, 17) für die Ortsmessung über ein Ortsmessunqs-, Steuer- und Auslesemodul SAMO vorgesehen ist, wobei zur transversalen Kompensation ein Vergleichen einer in dem Ortsmessungs-, Steuer- und Auslesemodul SAMO eines Kontrollsystems abqelegten Information über die Sollposition einer Bestrahlungsplanung mit der gemessenen Istposition der Strahlposition aus dem ortsempfindlichen Detektor in Echtzeit unter Berücksichtigung der erfassten transversalen Bewegungskomponente des Zielvolumens (1) möglich ist.

31. Vorrichtung nach einem der Ansprüche 13 bis 28 und 30, **dadurch gekennzeichnet, dass** die Rasterscanvorrichtung (3) Scannermagnet-Netzgeräte für die Ortskorrektur transversal in X- und Y-Richtung aufweist und der Tiefenmodulator (6) für die longitudinale Tiefenkorrektur vorgesehen ist.

32. Vorrichtung nach Anspruch 30, **dadurch gekennzeichnet, dass** eine schnelle Strahlabschaltung von dem Ortsmessung-, Steuer- und Auslesemodul SAMO des ortsempfindlichen Detektors in Echtzeit und/oder dem Bewegungsmessungs-, Steuer- und Auslesemodul SAMB des Tiefenmodulators (6) möglich ist, falls die Differenz zwischen einem Messwert und einem Sollwert der transversalen Strahlposition und/oder der longitudinalen Eindringtiefe w eine in der Fohtzeit-Software der Steuer- und Auslesemodule SAMO und/oder SAMB einstellbare Schwelle übersteigt.

## Claims

1. Apparatus for compensation of three-dimensional movements of a target volume (1) on a patient support apparatus (2) during ion beam irradiation using a raster scanning apparatus (3),
wherein the compensation apparatus comprises:
- a position location and tracking system (4) which detects the three-dimensional movements of the target volume (1), and
- a depth modulator (6) which re-adjusts the depth of penetration w of the ion beam (5),
- the raster scanning apparatus (3), which deflects the ion beam (5) transversely and which is in operative connection with a location measurement, control and read-out module (SAMO) and a module for changing the beam excursion (SAMS),
**characterised in that**
the position location and tracking system (4) is in operative connection with a movement measurement, control and read-out module SAMB;
and the depth modulator (6) is in operative connection with the movement measurement, control and read-out module SAMB, and the movement measurement, control and read-out module (SAMB) comprising a microprocessor having a memory,
and the memory comprising data of a model of a structure of healthy tissue that covers the target volume (1) in the upstream direction of the beam,
and the microprocessor comprising computational components which break down the detected movements of the target volume (1) vectorially into longitudinal and transverse components relative to the ion beam (5) and which compare the longitudinal components with the stored model for correction of the depth of penetration w of the ion beam (5).

2. Apparatus according to claim 1, **characterised in that** the raster scanning apparatus (3) comprises two raster scanning magnets (7, 8), which deflect an ion beam (5) orthogonally in relation to a coupling-in direction into the raster scanning magnets (7, 8), in two directions, which are in turn arranged perpendicular to one another, for scanning the area of the target volume (1) slice-wise.

3. Apparatus according to claim 2, **characterised in that** the raster scanning magnets (7, 8) are controlled by fast-reacting power supply units.

4. Apparatus according to one of the preceding claims, **characterised in that** the apparatus comprises ion acceleration elements by means of which the energy of the ion beam (5) can be adjusted so that the target volume (1) can be irradiated slice-wise, staggered in terms of depth of penetration w.

5. Apparatus according to one of the preceding claims, **characterised in that** the depth modulator (6) comprises two ion-braking plates (9, 10) of wedge-shaped cross-section which cover the entire irradiation zone of the scanned ion beam (5).

6. Apparatus according to claim 5, **characterised in that** the ion-braking plates (9, 10) are mounted on linear motors (11, 12).

7. Apparatus according to claim 5, **characterised in that** the ion-braking plates (9, 10) are arranged on electromagnetically actuatable carriages.

8. Apparatus according to one of claims 5 to 7, **characterised in that** the ion-braking plates (9, 10) are displaceable relative to one another with their wedge-shaped cross-sections overlapping in the region of the ion beam (5).

9. Apparatus according to one of claims 1 to 8, **characterised in that** the position location and tracking system (4) has at least one precision video camera (13) and/or X-ray detection means and/or ultrasound detection means, which are in operative connection with an image evaluation unit in the movement measurement, control and read-out module SAMB.

10. Apparatus according to one of the preceding claims, **characterised in that** an ionisation chamber (14, 15) having a fast read-out for monitoring the intensity of the ion beam stream is arranged as a transmission counter in the beam path of the ion beam (5).

11. Apparatus according to claim 10, **characterised in that** the ionisation chamber (14, 15) is arranged between the raster scanning apparatus (3) and the depth modulator (6).

12. Apparatus according to claim 11, **characterised in that** a multiwire proportional chamber (16, 17) is arranged as a location-sensitive detector in the beam direction upstream of the depth modulator (6).

13. Apparatus for modifying the depth of penetration of an ion beam in dependence upon movement of a patient on a patient support apparatus of a therapy facility, comprising
- a position location and tracking system (4) for monitoring movements of the patient,
- a depth modulator for adjusting the depth of penetration of the ion beam into the patient, and
- a movement measurement and control unit which is connected to the position location and tracking system (4) and to the depth modulator and which receives information relating to the movement of the patient from the position location and tracking system (4) and controls the depth modulator for modifying the depth of penetration, **characterised in that** the movement measurement and control unit comprises a microprocessor having a memory, and the memory comprising data of a model of a structure of healthy tissue that covers the target volume in the upstream direction of the beam, and the microprocessor, with the aid of the model and the information relating to the movement of the patient, so controlling the depth modulator that the depth of penetration of the ion beam is adjusted to a target volume element in the patient irrespective of the movement of the patient, especially irrespective of the movement of the healthy tissue relative to the target volume.

14. Apparatus according to claim 13, **characterised in that** the energy absorption of the tissue that the beam passes through and, as a result, the change in the range of the ion beam in dependence upon the tissue that the beam passes through can be calculated from the model.

15. Apparatus according to claim 13 or 14, **characterised in that** the tissue through which the beam is to pass can be determined from the information relating to the movement of the patient and the model.

16. Apparatus according to one of claims 13 to 15, **characterised in that** the model correlates changes in the electron density distribution in the healthy tissue, especially obtained by means of multidimensional projection radiographs or from time-resolved CT data sets, with movement states of the body.

17. Apparatus according to one of claims 13 to 16, **characterised in that** the depth modulator for modifying the depth of penetration comprises an apparatus for modifying the kinetic energy of the ions.

18. Apparatus according to one of claims 13 to 17, **characterised in that** the apparatus additionally comprises means of obtaining location information relating to the location of the ion beam relative to the patient, the movement measurement and control unit so controlling a raster scanning apparatus on the basis of the location information together with the aid of the model and the information relating to the movement of the patient that the ion beam follows a movement of the target volume in a transverse direction to the ion beam.

19. Apparatus according to one of claims 13 to 18, **characterised in that** the raster scanning apparatus (3) comprises two raster scanning magnets (7, 8), which deflect an ion beam (5) orthogonally in relation to a coupling-in direction into the raster scanning magnets (7, 8), in two directions which are in turn arranged perpendicular to one another, for scanning the area of the target volume (1) slice-wise.

20. Apparatus according to claim 19, **characterised in that** the raster scanning magnets (7, 8) are controlled by fast-reacting power supply units.

21. Apparatus according to one of claims 13 to 20, **characterised in that** the apparatus comprises ion acceleration elements by means of which the energy of the ion beam (5) can be adjusted so that the target volume (1) can be irradiated slice-wise, staggered in terms of depth of penetration w.

22. Apparatus according to one of claims 13 to 21, **characterised in that** the depth modulator (6) comprises two ion-braking plates (9, 10) of wedge-shaped cross-section which cover the entire irradiation zone of the scanned ion beam (5).

23. Apparatus according to claim 22, **characterised in that** the ion-braking plates (9, 10) are mounted on linear motors (11, 12).

24. Apparatus according to claim 22, **characterised in that** the ion-braking plates (9, 10) are arranged on electromagnetically actuatable carriages.

25. Apparatus according to one of claims 22 to 24, **characterised in that** the ion-braking plates (9, 10) are displaceable relative to one another with their wedge-shaped cross-sections overlapping in the region of the ion beam (5).

26. Apparatus according to one of claims 13 to 25, **characterised in that** the position location and tracking system (4) has at least one precision video camera (13) and/or X-ray detection means and/or ultrasound detection means, which are in operative connection with an image evaluation unit in the movement measurement, control and read-out module SAMB.

27. Apparatus according to one of claims 13 to 26, **characterised in that** a multiwire proportional chamber (16, 17) is arranged as a location-sensitive detector in the beam direction upstream of the depth modulator (6).

28. Apparatus according to one of claims 13 to 27, **characterised in that** the apparatus for detecting the structure of the healthy tissue covering the target volume in the upstream direction of the beam comprises an X-ray device and/or an ultrasound detection device for use in the preliminaries to and during ion beam irradiation.

29. Apparatus according to one of claims 13 to 28, **characterised in that** the raster scanning magnets (7, 8) comprise scanner magnet current power supply units for horizontal and vertical correction by means of control and read-out modules (SAMS) for the raster scanning magnets (7, 8) .

30. Apparatus according to one of claims 13 to 29, **characterised in that** a multiwire proportional chamber (16, 17) for location measurement, is provided by way of a location measurement, control and read-out module SAMO, it being possible, for the purpose of transverse compensation, to compare information stored in the location measurement, control and read-out module SAMO of a supervisory control system relating to the desired position of an irradiation plan with the measured actual position of the beam position from the location-sensitive detector in real time taking into account the detected transverse movement component of the target volume (1).

31. Apparatus according to one of claims 13 to 28 and 30, **characterised in that**, the raster scanning apparatus (3) comprises scanner magnet power supply units for the location correction in the transverse X and Y directions and the depth modulator (6) is provided for the longitudinal depth correction.

32. Apparatus according to claim 30, **characterised in that** fast shut-down of the beam by the location measurement, control and read-out module SAMO of the location-sensitive detector in real time and/or by the movement measurement, control and read-out module SAMB of the depth modulator (6) is possible, if the difference between a measured value and a desired value of the transverse beam position and/or of the longitudinal depth of penetration w exceeds a threshold that can be set in the real-time software of the control and read-out modules SAMO and/or SAMB.

## Revendications

1. Dispositif de compensation de mouvements tridimensionnels d'un volume cible (1) sur un dispositif de positionnement de patient (2) pendant une irradiation par faisceau ionique avec un dispositif à balayage par ligne (3), le dispositif de compensation comprenant :
- un système de positionnement et de poursuite (4) qui saisit les mouvements tridimensionnels du volume cible (1), et
- un modulateur de profondeur (6) qui régule la profondeur de pénétration w du faisceau ionique (5),
- un dispositif de balayage en ligne (3) qui dévie le faisceau ionique (5) transversalement et est en relation active avec un module de mesure sur site, de commande et de sélection (SAMO) et avec un module de modification de la déviation du rayon (SAMS),
**caractérisé en ce que**
le système de positionnement et de poursuite (4) est en relation active avec un module de mesure de mouvement, de commande et de sélection SAMB et **en ce que** le modulateur de profondeur (6) est en relation active avec le module de mesure de mouvement, de commande et de sélection SAMB et le module de mesure de mouvement, de commande et de sélection (SAMB) présente un microprocesseur à mémoire et la mémoire présente des données d'un modèle d'une structure d'un tissu sain qui couvre le volume cible (1) en amont du rayon et dans lequel le microprocesseur présente des composantes de calcul qui séparent les mouvements saisis du volume cible (1) sur le plan vectoriel en parties longitudinales et transversales par rapport au faisceau ionique (5) et qui compensent les parties longitudinales avec le modèle enregistré pour correction de la profondeur de pénétration w du faisceau ionique (5).

2. Dispositif selon la revendication 1, **caractérisé en ce que** le dispositif de balayage en ligne (3) présente deux aimants de balayage en ligne (7, 8) qui dévient un faisceau ionique (5) sur le plan orthogonal par rapport à un dispositif de couplage dans les aimants de balayage en ligne (7, 8) en deux directions qui sont elles-mêmes perpendiculaires l'une par rapport à l'autre, en un balayage de surface circulaire du volume cible (1).

3. Dispositif selon la revendication 2, **caractérisé en ce que** les aimants de balayage en ligne (7, 8) sont commandés par un appareil d'alimentation à la vitesse de la réaction.

4. Dispositif selon l'une des revendications précédentes, **caractérisé en ce que** le dispositif présente des éléments d'accélération ionique avec lesquels l'énergie du faisceau ionique (5) peut être ajustée de sorte que le volume cible (1) puisse être irradié progressivement de façon circulaire dans la profondeur de pénétration w.

5. Dispositif selon l'une des revendications précédentes, **caractérisé en ce que** le modulateur de profondeur (6) présente deux plaques de ralentissement d'ions (9, 10) de section conique qui couvrent le champ d'irradiation total du faisceau ionique balayé (5).

6. Dispositif selon la revendication 5, **caractérisé en ce que** les plaques de ralentissement d'ions (9, 10) sont montées sur des moteurs linéaires (11, 12).

7. Dispositif selon la revendication 5, **caractérisé en ce que** les plaques de ralentissement d'ions (9, 10) sont disposées sur des chariots pouvant être commandé de façon électromagnétique.

8. Dispositif selon l'une des revendications 5 à 7, **caractérisé en ce que** les plaques de ralentissement des ions (9, 10) peuvent être déplacées avec leur section conique de façon à se chevaucher l'une l'autre dans la zone du faisceau ionique (5).

9. Dispositif selon l'une des revendications 1 à 8, **caractérisé en ce que** le système de positionnement et de poursuite (4) présente au moins une caméra vidéo de précision (13) et/ou un moyen de saisie par rayon X et/ou un moyen de saisie par échographie qui sont raccordés activement avec une unité d'évaluation d'image dans le module de mesure de mouvement, de commande et de sélection SAMB.

10. Dispositif selon l'une des revendications précédentes, **caractérisé en ce qu'**une chambre d'ionisation en (14, 15) avec une sélection rapide pour la surveillance de l'intensité du courant de faisceau ionique en tant que compteur de transmission est disposée dans le parcours du faisceau ionique (5).

11. Dispositif selon la revendication 10, **caractérisé en ce que** la chambre d'ionisation (14, 15) est disposée entre le dispositif de balayage en ligne (3) et le modulateur de profondeur (6).

12. Dispositif selon la revendication 11, **caractérisé en ce qu'**une chambre proportionnelle multifils (16, 17) est disposée en tant que détecteur sensible sur site en amont du courant du modulateur de profondeur (6).

13. Dispositif d'adaptation de la profondeur de pénétration d'un faisceau ionique en fonction d'un mouvement d'un patient sur un dispositif de positionnement du patient d'une unité de traitement comprenant
- un système de positionnement et de poursuite (4), pour surveiller les mouvements du patient,
- un modulateur de profondeur pour réguler la profondeur de pénétration du faisceau ionique dans le patient, et
- une unité de mesure de mouvement et de commande qui est reliée au système de positionnement et de poursuite (4) et au modulateur de profondeur et obtient des informations du système de positionnement et de poursuite (4) par le mouvement du patient et commande le modulateur de profondeur pour ajuster la profondeur de pénétration,
**caractérisé en ce que** l'unité de mesure de mouvement et de commande présente un microprocesseur comportant une mémoire et dans lequel la mémoire présente des données d'un modèle d'une structure d'un tissu sain qui couvre le volume cible (1) en amont du rayon et dans lequel le microprocesseur, à l'aide du modèle et des informations sur le mouvement du patient, commande le modulateur de profondeur de telle sorte que la profondeur de pénétration du faisceau ionique soit ajustée indépendamment du mouvement du patient, en particulier indépendamment du mouvement du tissu sain par rapport au volume cible, sur un élément de volume cible du patient.

14. Dispositif selon la revendication 13, **caractérisé en ce que**, à partir du modèle, l'absorption d'énergie du tissu irradié et ainsi, la modification de la portée du faisceau ionique peuvent être calculées en fonction des tissus irradiés.

15. Dispositif selon la revendication 13 ou 14, **caractérisé en ce qu'**à partir des informations sur le mouvement du patient et du modèle, on peut déterminer le tissu à irradier.

16. Dispositif selon l'une des revendications 13 à 15, **caractérisé en ce que** le modèle est corrélé à des modifications dans la répartition de la densité d'électrons dans le tissu sain, en particulier par le biais de clichés de projection radiographique multidimensionnels ou obtenus de données de CT résolues dans le temps, avec des états de mouvements du corps.

17. Dispositif selon l'une des revendications 13 à 16, **caractérisé en ce que** le modulateur de profondeur présente, pour la modification de la profondeur de pénétration, un dispositif de modification de l'énergie cinétique d'ions.

18. Dispositif selon l'une des revendications 13 à 17, **caractérisé en ce que** le dispositif présente en outre des moyens d'obtention d'informations de position, sur la position du faisceau ionique par rapport au patient, dans lequel l'unité de mesure de mouvement et de commande, commande à partir des informations de position conjointement à l'aide du modèle et des informations sur le mouvement du patient, un dispositif de balayage en ligne de telle sorte que le faisceau ionique suive un mouvement du volume cible transversalement par rapport au faisceau ionique.

19. Dispositif selon l'une des revendications 13 à 18, **caractérisé en ce que** le dispositif de balayage en ligne (3) présente deux aimants de balayage en ligne (7, 8) qui dévient un faisceau ionique (5) sur le plan orthogonal par rapport à un dispositif de couplage dans les aimants de balayage en ligne (7, 8) dans deux directions qui sont elles-mêmes perpendiculaires l'une par rapport à l'autre, en un balayage de surface circulaire du volume cible (1).

20. Dispositif selon la revendication 19, **caractérisé en ce que** les aimants de balayage en ligne (7, 8) sont commandés par un appareil d'alimentation à la vitesse de la réaction.

21. Dispositif selon l'une des revendications 13 à 20, **caractérisé en ce que** le dispositif présente des éléments d'accélération ionique avec lesquels l'énergie du faisceau ionique (5) peut être ajustée de sorte que le volume cible (1) puisse être irradié progressivement de façon circulaire dans la profondeur de pénétration w.

22. Dispositif selon l'une des revendications 13 à 21, **caractérisé en ce que** le modulateur de profondeur (6) présente deux plaques de ralentissement d'ions (9, 10) de section conique qui couvrent le champ d'irradiation total du faisceau ionique balayé (5).

23. Dispositif selon la revendication 22, **caractérisé en ce que** les plaques de ralentissement d'ions (9, 10) sont montées sur des moteurs linéaires (11, 12) .

24. Dispositif selon la revendication 22, **caractérisé en ce que** les plaques de ralentissement d'ions (9, 10) sont disposées sur des chariots pouvant être commandé de façon électromagnétique.

25. Dispositif selon l'une des revendications 22 à 24, **caractérisé en ce que** les plaques de ralentissement des ions (9, 10) peuvent être déplacées avec leur section conique de façon à se chevaucher l'une l'autre dans la zone du faisceau ionique (5).

26. Dispositif selon l'une des revendications 13 à 25, **caractérisé en ce que** le système de positionnement et de poursuite (4) présente au moins une caméra vidéo de précision (13) et/ou un moyen de saisie par rayon X et/ou un moyen de saisie par échographie qui sont raccordés activement avec une unité d'évaluation d'image dans le module de mesure de mouvement, de commande et de sélection SAMB.

27. Dispositif selon l'une des revendications 13 à 26, **caractérisé en ce qu'**une chambre proportionnelle multifils (16, 17) est disposée en tant que détecteur sensible sur site en amont du courant du modulateur de profondeur (6).

28. Dispositif selon l'une des revendications 13 à 27, **caractérisé en ce que** le dispositif de saisie de la structure du tissu sain qui couvre le volume cible en amont du rayon présente un appareil à rayons X et/ou un appareil échographique pour l'utilisation en première ligne et pendant une irradiation par faisceau ionique.

29. Dispositif selon l'une des revendications 13 à 28, **caractérisé en ce que** les aimants de balayage en ligne (7, 8) présentent des appareils d'alimentation à aimant de balayage pour une correction horizontale et verticale sur le module de commande et de sélection (SAMS) pour les aimants de balayage en ligne (7, 8).

30. Dispositif selon l'une des revendications 13 à 29, **caractérisé en ce qu'**est prévue une chambre proportionnelle multifils (16, 17) pour la mesure sur site par le biais d'un module de mesure sur site, de commande et de sélection SAMO, dans lequel, pour la compensation transversale, une comparaison d'informations déposées dans le module de mesure sur site, de commande et de sélection d'un système de contrôle est possible par le biais de la position théorique d'une planification de rayonnement avec la position réelle mesurée de la position de rayonnement du détecteur sensible au site en temps réel en tenant compte des composants de mouvement transversaux saisis du volume cible (1).

31. Dispositif selon l'une des revendications 13 à 28 et 30, **caractérisé en ce que** le dispositif de balayage en ligne (3) présente un appareil d'alimentation à aimant de balayage pour la correction sur site dans les directions X et Y transversalement, et le modulateur de profondeur (6) est prévu pour la correction en profondeur longitudinale.

32. Dispositif selon la revendication 30, **caractérisé en ce qu'**un arrêt rapide du faisceau du module de mesure sur site, de commande et de sélection SAMO du détecteur sensible au site en temps réel et/ou du module de mesure de mouvement, de commande et de sélection SAMB du modulateur de profondeur (6) est possible si la différence entre une mesure et une valeur théorique de la position de rayon transversale et/ou de la profondeur de pénétration longitudinale w dépasse un seuil ajustable dans le logiciel en temps réel du module de commande et de sélection SAMO et/ou SAMB.
